(19) Europäisches Patentamt
European Patent Office
Office européen des brevets

(11) Veröffentlichungsnummer: **0 131 692**
**A1**

# (12) EUROPÄISCHE PATENTANMELDUNG

(21) Anmeldenummer: **84103747.6**

(22) Anmeldetag: **05.04.84**

(51) Int. Cl.[4]: **A 01 N 37/38**
A 01 N 37/36, C 07 C 149/415
A 01 N 43/40, A 01 N 43/10
C 07 D 213/57, C 07 D 333/24

(30) Priorität: **16.06.83 DE 3322281**

(43) Veröffentlichungstag der Anmeldung:
**23.01.85 Patentblatt 85/4**

(84) Benannte Vertragsstaaten:
**BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Skötsch, Carlo Dr.**
**Kaiserin-Augusta-Allee 83**
**D-1000 Berlin 10(DE)**

(72) Erfinder: **Joppien, Hartmut, Dr.**
**Juttastrasse 18**
**D-1000 Berlin 37(DE)**

(72) Erfinder: **Pieroh, Ernst Albrecht**
**Im Fischgrund 18**
**D-1000 Berlin 28(DE)**

## (54) Alpha-Alkylthiozimtsäurenitrile zur Bekämpfung von Schadorganismen.

(57) Die Erfindung betrifft ein Mittel zur Bekämpfung von Schadorganismen, gekennzeichnet durch einen Gehalt an mindestens einem α-Alkylthiozimtsäurenitril der allgemeinen Formel

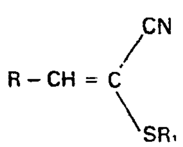
, I

in der

R einen gegebenenfalls ein- oder mehrfach, gleich oder verschieden durch Halogen, $(C_1-C_4)$-Alkyl, $(C_1-C_4)$-Alkoxy, $(C_1-C_4)$-Alkylthio oder Trifluormethyl substituierten aromatischen Kohlenwasserstoffrest und

$R_1$ einen gradkettigen $(C_1-C_4)$-Alkylrest oder den Cyanäthylrest bedeuten, sowie ein Verfahren zur Herstellung dieser Verbindungen.

EP 0 131 692 A1

Croydon Printing Company Ltd

Die Erfindung betrifft ein neues Mittel enthaltend α-Alkylthiozimtsäurenitrile zur Bekämpfung von Schadorganismen sowie ein Verfahren zur Herstellung dieser Verbindungen.

3-Alkylthio-2-aryl-acrylnitrile mit herbizider Wirkung sind bereits bekannt (US-PS 3.828.091) Zur Bekämpfung von Schadorganismen sind diese Verbindungen nicht geeignet.

Aufgabe der vorliegenden Erfindung ist die Zurverfügungstellung eines Mittels, welches eine Bekämpfung von Schadorganismen ermöglicht.

Diese Aufgabe wird erfindungsgemäß durch ein Mittel gelöst, das gekennzeichnet ist durch einen Gehalt an mindestens einem α-Alkylthiozimtsäurenitril der allgemeinen Formel

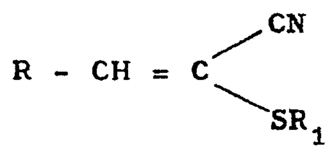 , I

in der

R einen gegebenenfalls ein- oder mehrfach, gleich oder verschieden durch Halogen, $(C_1-C_4)$-Alkyl, $(C_1-C_4)$-Alkoxy, $(C_1-C_4)$-Alkylthio oder Trifluormethyl substituierten aromatischen Kohlenwasserstoffrest und

$R_1$ einen gradkettigen $(C_1-C_4)$-Alkylrest oder den Cyanäthylrest bedeuten.

Die erfindungsgemäßen Mittel eignen sich vorteilhafterweise zur Bekämpfung von pflanzenfressenden Schädlingspopulationen aus den Insektenordnungen der Lepidopteren und Coleopteren. Ihre Wirkung entfalten die Mittel dabei besonders auf die frisch abgelegten Eier oder Eigelege der Schädlinge.

Die überraschend gute nematizide Wirkung der beschriebenen neuen Mittel umfaßt auch larvizide und ovizide Wirkungen bei Wurzelgallennematoden sowie Schlüpfhemmeffekte bei cystenbildenden Wurzelnematoden (Meloidogyne- und Heterodera-Arten),

-3-

Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin
Vorstand Dr Herbert Asmis Dr Christian Bruhn Dr Heinz Hannse, Horst Kramp, Dr Klaus Pohle, Dr Horst Witzel · Vorsitzender des Aufsichtsrats: Hans-Jürgen Hamann Sitz der Gesellschaft Berlin und Bergkamen Handelsregister AG Charlottenburg 93 HRB 283 und AG Kamen HRB 0061 · Berliner Commerzbank AG, Berlin, Konto-Nr 108700600 Bankleitzahl 100 400 00 Berliner Handels- und Frankfurter Bank, Berlin, Konto-Nr 70045224, Bankleitzahl 100 202 00 · Deutsche Bank Berlin AG, Konto-Nr 2415008 Bankleitzahl 100 700 00 Postscheckamt Berlin West, Konto-Nr 1175-101, Bankleitzahl 100 100 10

worin sie sogar praxisbekannten Präparaten gleicher Wirkungsrichtung nach Vitro-Ergebnissen überlegen sind.

Von besonderem Vorteil ist es, daß die erfindungsgemäßen Mittel zur Beeinträchtigung der Resistenzbildung von Schadorganismenpopulationen und für moderne Bekämpfungsmethoden verwendet werden können.

Die Aufwandmenge richtet sich nach der Art der zu bekämpfenden Schädlinge und beträgt im allgemeinen von 0,5 bis 20 kg/ha.

Von den durch die allgemeine Formel I gekennzeichneten Verbindungen zeichnen sich durch eine gute Wirkung beispielsweise diejenigen aus, bei denen

R Phenyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Jodphenyl, 3-Jodphenyl, 4-Jodphenyl, 2,4-Difluorphenyl, 2,6-Difluorphenyl, Pentafluorphenyl, 2,4-Dichlorphenyl, 3,4-Dichlorphenyl, 2-Chlor-6-fluorphenyl, 2-Methylphenyl, 3-Methylphenyl, 4-Methylphenyl, 2-Methoxyphenyl, 3-Methoxyphenyl, 4-Methoxyphenyl, 2-Methylthiophenyl, 3-Methylthiophenyl, 4-Methylthiophenyl, 3-Trifluormethyl, 4-Trifluormethyl, 2-Pyridyl, 3-Pyridyl, 4-Pyridyl, 2-Thienyl, 3-Thienyl oder 1-Naphthyl und

$R_1$ Methyl, Äthyl, Propyl, Butyl oder Cyanäthyl bedeuten.

Eine hervorragende insektizide und ovizide Wirkung zeigen zum Beispiel die folgenden Verbindungen:
α-Methylthio-zimtsäurenitril und
α-Methylthio-2-fluorzimtsäurenitril

Als nematizid besonders wirksame Verbindungen sind beispielsweise zu nennen:

-4-

Postanschrift: Schering Aktiengesellschaft, Postfach 65 03 11, D-1000 Berlin 65 · Fur Besucher Berlin-Wedding, Mullerstraße 170-178 Telegramme Scheringchemie Berlin
Vorstand Dr. Herbert Asmis Dr. Christian Bruhn Dr. Heinz Hannse Horst Kramp. Dr. Klaus Pohle Dr. Horst Witzel Vorsitzender des Aufsichtsrats Hans-Jürgen Hamann
Sitz der Gesellschaft Berlin und Bergkamen Handelsregister AG Charlottenburg 93 HRB 283 und AG Kamen HRB 0061 Berliner Commerzbank AG, Berlin, Konto-Nr 108700600 Bankleitzahl 100 400 00 Berliner Handels- und Frankfurter Bank, Berlin, Konto-Nr. 70045224, Bankleitzahl 100 202 00 · Deutsche Bank Berlin AG, Konto-Nr 2415008 Bankleitzahl 100 700 00 Postscheckamt Berlin West Konto-Nr 11 75-101 Bankleitzahl 100 100 10

α-Methylthio-4-chlorzimtsäurenitril
α-Methylthio-4-bromzimtsäurenitril
α-Methylthio-4-fluorzimtsäurenitril

Die erfindungsgemäß zu verwendenden Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Sofern eine Verbreiterung des Wirkungsspektrums beabsichtigt ist, können auch andere Biozide zugesetzt werden. Außerdem können auch nicht phytotoxische Mittel angewendet werden, die mit Bioziden eine synergistische Wirkungssteigerung ergeben können, wie unter anderem Netzmittel, Emulgatoren, Lösungsmittel und ölige Zusätze.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Netz-, Haft-, Emulgier- und/oder Dispergierhilfsmitteln angewandt.

Geeignete flüssige Trägerstoffe sind zum Beispiel Wasser, aliphatische und aromatische Kohlenwasserstoffe, wie Benzol, Toluol, Xylol, Cyclohexanon, Isophoron, Dimethylsulfoxyd, weiterhin Mineralölfraktionen und Pflanzenöle.

-5-

Als feste Trägerstoffe eignen sich Mineralerden, zum Beispiel Tonsil, Silicagel, Talkum, Kaolin, Attapulgit, Kalkstein, Kieselsäure und pflanzliche Produkte, zum Beispiel Mehle.

An oberflächenaktiven Stoffen sind zu nennen: zum Beispiel Calciumligninsulfonat, Polyoxyäthylenalkylphenyl-äther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Sofern die Wirkstoffe zur Saatgutbeizung Verwendung finden sollen, können auch Farbstoffe zugemischt werden, um dem gebeizten Saatgut eine deutlich sichtbare Färbung zu geben.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 Liter/ha. Eine Anwendung der Mittel im sogenannten "Low-Volume-" oder "Ultra-Low-Volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:

A. <u>Spritzpulver</u>

a) 40 Gewichtsprozent Wirkstoff
   25 Gewichtsprozent Tonmineralien
   20 Gewichtsprozent Kieselsäure
   10 Gewichtsprozent Zellpech



Postanschrift: Schering Aktiengesellschaft, Postfach 65 03 11, D-1000 Berlin 65 · Fur Besucher Berlin-Wedding, Mullerstraße 170-178 Telegramme: Scheringchemie Berlin
Vorstand Dr Herbert Asmis Dr Christian Bruhn, Dr Heinz Hannse Horst Kramp, Dr. Klaus Pohle, Dr Horst Witzel Vorsitzender des Aufsichtsrats: Hans-Jürgen Hamann Sitz der Gesellschaft Berlin und Bergkamen Handelsregister AG Charlottenburg 93 HRB 283 und AG Kamen HRB 0061 Berliner Commerzbank AG. Berlin, Konto-Nr 108700600 Bankleitzahl 100 400 00 Berliner Handels- und Frankfurter Bank, Berlin, Konto-Nr. 70045224, Bankleitzahl 100 202 00 · Deutsche Bank Berlin AG, Konto-Nr 2415008 Bankleitzahl 100 700 00 Postscheckamt Berlin West Konto-Nr 11 75-101, Bankleitzahl 100 100 10

5 Gewichtsprozent oberflächenaktive Stoffe auf der Basis einer Mischung des Calciumsalzes der Ligninsulfonsäure mit Alkylphenolpolyglykoläthern

b) 25 Gewichtsprozent Wirkstoff
60 Gewichtsprozent Kaolin
10 Gewichtsprozent Kieselsäure
5 Gewichtsprozent oberflächenaktive Stoffe auf Basis des Natriumsalzes des N-Methyl-N-oleyl-taurins und des Calciumsalzes der Ligninsulfonsäure.

c) 10 Gewichtsprozent Wirkstoff
60 Gewichtsprozent Tonmineralien
15 Gewichtsprozent Kieselsäure
10 Gewichtsprozent Zellpech
5 Gewichtsprozent oberflächenaktive Stoffe auf Basis des Natriumsalzes des N-Methyl-N-oleyl-taurins und des Calciumsalzes der Ligninsulfonsäure.

B. <u>Paste</u>
45 Gewichtsprozent Wirkstoff
5 Gewichtsprozent Natriumaluminiumsilikat
15 Gewichtsprozent Cetylpolyglycoläther mit 8 Mol Äthylenoxid
2 Gewichtsprozent Spindelöl
10 Gewichtsprozent Polyäthylenglycol
23 Teile Wasser

C. <u>Emulsionkonzentrat</u>
25 Gewichtsprozent Wirkstoff
15 Gewichtsprozent Cyclohexanon
55 Gewichtsprozent Xylol
5 Gewichtsprozent Mischung von Nonylphenylpolyoxyäthylen oder Calciumdodecylbenzolsulfonat.



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand Dr Herbert Asmis Dr Christian Bruhn Dr Heinz Hannse Horst Kramp Dr Klaus Pohle Dr Horst Witzel Vorsitzender des Aufsichtsrats: Hans-Jürgen Hamann Sitz der Gesellschaft Berlin und Bergkamen Handelsregister AG Charlottenburg 93 HRB 283 und AG Kamen HRB 0061 Berliner Commerzbank AG, Berlin, Konto-Nr 108[illegible] Bankleitzahl 100 400 00 Berliner Handels- und Frankfurter Bank Berlin, Konto-Nr 70045224, Bankleitzahl 100 202 00 Deutsche Bank Berlin AG, Konto-Nr 241[illegible] Bankleitzahl 100 700 00 Postscheckamt Berlin West Konto-Nr 1175-101, Bankleitzahl 100 100 10

Die erfindungsgemäß zu verwendenden Verbindungen lassen sich in besonders vorteilhafter Weise herstellen, indem man aromatische Aldehyde der allgemeinen Formel

$$R - CHO \qquad II$$

mit Alkylthioacetonitrilen der allgemeinen Formel

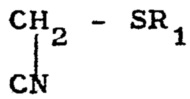

in Gegenwart von 18-crown-6 als Katalysator und unter Zusatz einer Base gegebenenfalls gelöst in einem Lösungsmittel kondensiert und das gewünschte Reaktionsprodukt in an sich bekannter Weise isoliert.

Im allgemeinen entstehen wegen der Doppelbindung Z, E-Isomere. Die Isomeren können gewünschtenfalls mit Hilfe herkömmlicher Methoden, wie fraktionierte Destillation oder Säulenchromatographie, getrennt werden.
Erfindungsgemäß können sowohl die Z, E-Isomeren-gemische als auch die einzelnen Z-beziehungsweise E-Komponenten, verwendet werden.

Die Kondensationsreaktion zwischen Aldehyd und Alkylthioacetonitril kann mit oder ohne Lösungsmittel durchgeführt werden. Als Lösungsmittel eignen sich im allgemeinen gegenüber den Reaktanden inerte Stoffe wie Alkohole, Äther, Acetonitril, Toluol und andere. Die Reaktion läuft im allgemeinen im Basischen ab, wobei Alkalihydroxyd in kleinen Mengen (ca. 0,1 Äquivalent) als Base und katalytische Mengen 18-Crown-6 zugesetzt werden.

Die erfindungsgemäß zu verwendenden α-Alkylthiozimtsäurenitrile sind im allgemeinen farblose bis gelblich gefärbte Flüssigkeiten von esterartigem Geruch. Sie lösen sich gut in organischen Lösungsmitteln, wie zum Beispiel Benzin, Xylol, Cyclohexanon, Aceton, Methylenchlorid, Chloroform, Äthanol, Acetonitril und Dimethylformamid. In Wasser sind sie zu weniger als 1% löslich.



**Postanschrift: Schering Aktiengesellschaft, Postfach 65 03 11, D-1000 Berlin 65** · Fur Besucher Berlin-Wedding Mullerstraße 170-178 · Telegramme Scheringchemie Berlin
Vorstand Dr Herbert Asmis Dr Christian Bruhn Dr Heinz Hannse, Horst Kramp, Dr Klaus Pohle, Dr Horst Witzel Vorsitzender des Aufsichtsrats Hans-Jurgen Hamann
Sitz der Gesellschaft Berlin und Bergkamen Handelsregister AG Charlottenburg 93 HRB 283 und AG Kamen HRB 0061 Berliner Commerzbank AG, Berlin, Konto-Nr 108700600 Bankleitzahl 100 400 00 Berliner Handels- und Frankfurter Bank, Berlin, Konto-Nr 70045224, Bankleitzahl 100 202 00 · Deutsche Bank Berlin AG, Konto-Nr 2415008 Bankleitzahl 100 700 00 Postscheckamt Berlin West Konto-Nr 1175-101, Bankleitzahl 100 100 10

Die folgenden Beispiele dienen zur Erläuterung der Herstellung der erfindungsgemäß zu verwendenden Verbindungen.

BEISPIEL 1

α-Methylthio-2-fluorzimtsäurenitril, Isomerengemisch

65,36 g (0,75 Mol) Methylthioacetonitril werden in 750 ml Tetrahydrofuran gelöst. Man gibt 5,6 g (0,1 Mol) Kaliumhydroxid und 0,3 g 18-Crown-6 hinzu. Bei 10°C werden innerhalb von 20 Minuten 93,08 g 2-Fluorbenzoldehyd in 250 ml Tetrahydrofuran zurgetropft. Man rührt 2 Stunden bei 10°C und läßt dann über Nacht stehen. Die Lösung wird filtriert, der Rückstand wird mit Tetrahydrofuran gewaschen. Man rotiert im Vakuum ein, nimmt das zurückbleibende Öl in 1 Liter Essigester auf und wäscht mit 2 mal je 500 ml verdünnter Natriumchloridlösung. Die Essigesterphase wird über Magnesiumsulfat getrocknet und nach dem Abfiltrieren im Vakuum einrotiert. Man destilliert den Rückstand im Ölpumpenvakuum.

$Kp_{0,1-0,2}$: 98-103°C $n_D^{20}$: 1,6155

Ausbeute: 105,1 g = 72,5 % der Theorie
Es liegt ein 60:40 E, Z-Gemisch vor.



Postanschrift: Schering Aktiengesellschaft, Postfach 65 03 11, D-1000 Berlin 65 · Für Besucher Berlin-Wedding Müllerstraße 170-178 · Telegramme: Scheringchemie
Vorstand Dr. Herbert Asmis Dr. Christian Bruhn Dr. Heinz Hannse Horst Kramp Dr. Klaus Pohle, Dr. Horst Witzel Vorsitzender des Aufsichtsrats: Hans-Jürg
Sitz der Gesellschaft Berlin und Bergkamen Handelsregister AG Charlottenburg 93 HRB 283 und AG Kamen HRB 0061 Berliner Commerzbank AG, B
108700600 Bankleitzahl 100 400 00 Berliner Handels- und Frankfurter Bank Berlin Konto-Nr 70045224. Bankleitzahl 100 202 00 · Deutsche Bank Be
2415008 Bankleitzahl 100 700 00 Postscheckamt Berlin West Konto-Nr 1175-101, Bankleitzahl 100 100 10

BEISPIEL 2

Trennung von Z, E- α-Methylthio-2-Fluorzimtsäurenitril in E-α-Methylthio-2-fluorzimtsäurenitril und Z-α-Methylthio-2-fluorzimtsäurenitril

Präparative HPLC-Chromatographie von 210 mg Z,E-α-Methylthio-2-fluorzimtsäurenitril mit Isococtan:Diisopropyläther = 1000 ml : 4 ml ergab:

115 mg E-α-Methylthio-2-fluorzimtsäurenitril

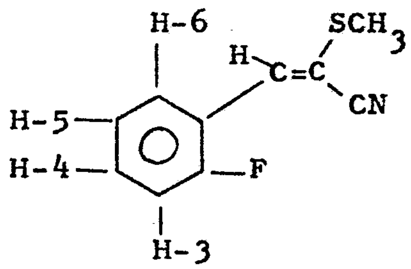

$^{1}$H-NMR: $SCH_3$: 2,5 ppm(s); H-3, H-4, H-5, H\C= : 6,9-7,5 ppm(m);
H-6 : 8,03 ppm(m) und

84 mg Z-α-Methylthio-2-Fluorzimtsäurenitril

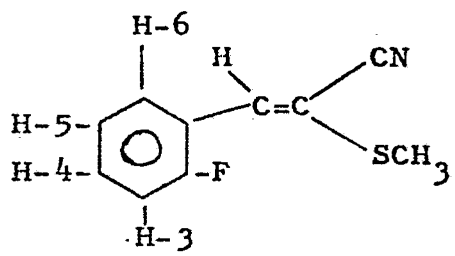

$^{1}$H-NMR: $SCH_3$: 2,57 ppm(s); H-3, H-4, H-5, H\C= : 6,9-7,5ppm(m);
H-6 : 7,78 ppm(m)



Postanschrift: Schering Aktiengesellschaft, Postfach 65 03 11, D-1000 Berlin 65 · Für Besucher Berlin-Wedding Müllerstraße 170-178 · Telegramme Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann · Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Wie im Beispiel 1 können folgende Verbindungen hergestellt werden.

| Beispiel Nr. | Name der Verbindungen | Physikalische Konstante |
|---|---|---|
| 3 | α-Ethylthio-zimtsäurenitril, Isomerengemisch | Kp 136-42°C/0,4 mm |
| 4 | α-Ethylthio-2-chlorzimtsäurenitril, Isomerengemisch | Kp 155-57°C/1 mm |
| 5 | α-Ethylthio-4-chlorzimtsäurenitril, Isomerengemisch | Kp 130-35°C/0,05 mm |
| 6 | α-Ethylthio-2,4-dichlorzimtsäurenitril, Isomerengemisch | Kp 125-30°C/0,05 mm |
| 7 | α-Ethylthio-3,4-dichlorzimtsäurenitril, Isomerengemisch | Kp 142-48°C/0,05 mm |
| 8 | 2-Ethylthio-3-(pyridin-2-yl)-acylnitril, Isomerengemisch | Kp 145-50°C/0,05 mm |
| 9 | 2-Ethylthio-3-(2-thienyl)-acrylnitril, Isomerengemisch | Kp 118-25°C/0,05 mm |
| 10 | α-Ethylthio-2-methylzimtsäurenitril, Isomerengemisch | Kp 115-22°C/0,05 mm |
| 11 | α-Ethylthio-3-chlorzimtsäurenitril, Isomerengemisch | Kp 125-30°C/0,05 mm |
| 12 | α-Methylthio-zimtsäurenitril, Isomerengemisch | Kp 130-32°C/0,4 mm |
| 13 | α-Methylthio-3-chlorzimtsäurenitril, Isomerengemisch | Kp 128-30°C/0,05 mm |
| 14 | α-Methylthio-2-methylzimtsäurenitril, Isomerengemisch | Kp 115-19°C/0,05 mm |
| 15 | α-Methylthio-4-zimtsäurenitril, Isomerengemisch | $n_D^{20}$: 1,6575 |
| 16 | α-Methylthio-2-chlorzimtsäurenitril, Isomerengemisch | $n_D^{20}$: 1,6401 |
| 17 | α-Methylthio-4-bromzimtsäurenitril, Isomerengemisch | $n_D^{20}$: 1,6780 |
| 18 | α-Methylthio-2-methoxyzimtsäurenitril, Isomerengemisch | $n_D^{20}$: 1,6365 |

-11-

Postanschrift Schering Aktiengesellschaft, Postfach 65 03 11, D-1000 Berlin 65 · Für Besucher Berlin-Wedding Müllerstraße 170-178 Telegramme Scheringchemie Berlin
Vorstand: Dr. Heribert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG Berlin, Konto-Nr. 108700600, Bankleitzahl 100 400 00 · Berliner Handels- und Frankfurter Bank Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175 101, Bankleitzahl 100 100 10

| Beispiel Nr. | Name der Verbindungen | Physikalische Konstante |
|---|---|---|
| 19 | α-Methylthio-4-fluorzimtsäurenitril, Isomerengemisch | $n_D^{20}$: 1,6165 |
| 20 | 2-Methylthio-3-(1-naphthyl)-acrylnitril, Isomerengemisch | $n_D^{20}$: 1,6950 |
| 21 | 2-Methylthio-3-(3-pyridyl)-acrylnitril, Isomerengemisch | $n_D^{20}$: 1,6405 |
| 22 | 2-Methylthio-3-(2-pyridyl)-acrylnitril, Isomerengemisch | $n_D^{20}$: 1,6240 |
| 23 | α-Methylthio-2,4-dichlorzimtsäurenitril, Isomerengemisch | Fp.: 65-66°C |
| 24 | α-Methylthio-3-trifluormethylzimtsäurenitril, Isomerengemisch | $n_D^{20}$: 1,5575 |
| 25 | 2-Chlor-6-fluor-α-methylthio-zimtsäurenitril | $n_D^{20}$: 1,6020 |
| 26 | α-Methylthio-4-trifluormethylzimtsäurenitril, Isomerengemisch | $n_D^{20}$: 1,5655 |
| 27 | α-Ethylthio-2-fluorzimtsäurenitril, Isomerengemisch | $n_D^{20}$: 1,6020 |
| 28 | α-Methylthio-4-methylzimtsäurenitril, Isomerengemisch | $n_D^{20}$: 1,6345 |

Die folgenden Ausführungsbeispiele dienen zur Erläuterung der Anwendungsmöglichkeiten der erfindungsgemäßen Mittel, die in Form der oben angeführten Zubereitungen erfolgte.



Postanschrift Schering Aktiengesellschaft, Postfach 65 03 11, D-1000 Berlin 65 · Fur Besucher Berlin-Wedding Mullerstraße 170-178 Telegramme Scheringchemie Berlin
Vorstand Dr Herbert Asmis Dr Christian Bruhn Dr Heinz Hannse Horst Kramp Dr Klaus Pohle Dr Horst Witzel Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175-101 Bankleitzahl 100 100 10

BEISPIEL 29

Ovizide Wirkung auf Eigelege der Ägyptischen Baumwolleule (Spodoptera littoralis)

Die erfindungsgemäß zu verwendenden Verbindungen wurden als wäßrige Suspensionen mit der Wirkstoffkonzentration 0,1 % eingesetzt. In diese Wirkstoffzubereitung wurden 1 Tag alte Eigelege, die von befruchteten Falterweibchen auf Filterpapier abgesetzt worden waren, bis zur völligen Benetzung getaucht und zur Auswertung für 4 Tage in geschlossenen Petrischalen deponiert. Kriterium für die Wirkungsbeurteilung war die prozentuale Schlupfverhinderung im Vergleich zu unbehandelten Eigelegen.

Die erzielten Ergebnisse sind in nachstehender Tabelle zusammengefaßt.

| Verbindungen | Wirkstoffkonzentration in % | Schlupfverhinderung in % |
|---|---|---|
| α-Ethylthio-zimtsäurenitril, Isomerengemisch | 0,1 | 100 |
| α-Ethylthio-2-chlorzimtsäurenitril, Isomerengemisch | 0,1 | 100 |
| α-Ethylthio-4-chlorzimtsäurenitril, Isomerengemisch | 0,1 | 100 |
| α-Ethylthio-2,4-dichlorzimtsäurenitril, Isomerengemisch | 0,1 | 100 |
| α-Ethylthio-3,4-dichlorzimtsäurenitril, Isomerengemisch | 0,1 | 100 |
| 2-Ethylthio-3-(pyridin-2-yl)-acylnitril, Isomerengemisch | 0,1 | 100 |
| 2-Ethylthio-3-(2-thienyl)-acrylnitril, Isomerengemisch | 0,1 | 100 |
| α-Ethylthio-2-methylzimtsäurenitril, Isomerengemisch | 0,1 | 100 |
| α-Ethylthio-3-chlorzimtsäurenitril, Isomerengemisch | 0,1 | 100 |
| α-Methylthio-zimtsäurenitril, Isomerengemisch | 0,1 | 100 |

-13-

Postanschrift: Schering Aktiengesellschaft, Postfach 65 03 11, D-1000 Berlin 65 · Fur Besucher Berlin-Wedding Mullerstraße 170-178 Telegramme Scheringchemie Berlin
Vorstand Dr. Herbert Asmis Dr. Christian Bruhn Dr. Heinz Hannse Horst Kramp Dr. Klaus Pohle Dr. Horst Witzel Vorsitzender des Aufsichtsrats Hans-Jurgen Hamann Sitz der Gesellschaft Berlin und Bergkamen Handelsregister AG Charlottenburg 93 HRB 283 und AG Kamen HRB 0061 Berliner Commerzbank AG Berlin Konto-Nr 1087 006 00 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto Nr 1175 101 Bankleitzahl 100 100 10

| Verbindungen | Wirkstoffkonzentration in % | Schlupfverhinderung in % |
|---|---|---|
| α-Methylthio-3-chlorzimtsäurenitril, Isomerengemisch | 0,1 | 100 |
| α-Methylthio-2-methylzimtsäurenitril, Isomerengemisch | 0,1 | 100 |
| α-Methylthio-4-zimtsäurenitril, Isomerengemisch | 0,1 | 100 |
| α-Methylthio-2-chlorzimtsäurenitril, Isomerengemisch | 0,1 | 100 |
| α-Methylthio-4-bromzimtsäurenitril, Isomerengemisch | 0,1 | 100 |
| α-Methylthio-2-fluorzimtsäurenitril, Isomerengemisch | 0,1 | 100 |
| α-Methylthio-2-methoxyzimtsäurenitril, Isomerengemisch | 0,1 | 80 |
| α-Methylthio-4-fluorzimtsäurenitril, Isomerengemisch | 0,1 | 100 |
| 2-Methylthio-3-(1-naphthyl)-acrylnitril, Isomerengemisch | 0,1 | 100 |
| 2-Methylthio-3-(3-pyridyl)-acrylnitril, Isomerengemisch | 0,1 | 100 |
| α-Methylthio-2,4-dichlorzimtsäurenitril, Isomerengemisch | 0,1 | 100 |
| α-Methylthio-3-trifluormethylzimtsäurenitril, Isomerengemisch | 0,1 | 100 |
| 2-Chlor-6-fluor-α-methylthio-zimtsäurenitril | 0,1 | 100 |
| α-Methylthio-4-trifluormethylzimtsäurenitril, Isomerengemisch | 0,1 | 100 |
| α-Ethylthio-2-fluorzimtsäurenitril, Isomerengemisch | 0,1 | 100 |

-14-

Postanschrift Schering Aktiengesellschaft, Postfach 65 03 11, D-1000 Berlin 65 · Für Besucher Berlin-Wedding Müllerstraße 170-178 Telegramme Scheringchemie Berlin
Vorstand Dr. Herbert Asmis Dr. Christian Bruhn Dr. Heinz Hannse Horst Kramp Dr. Klaus Pohle Dr. Horst Witzel Vorsitzender des Aufsichtsrats Hans-Jürgen Hamann Sitz der Gesellschaft Berlin und Bergkamen Handelsregister AG Charlottenburg 93 HRB 283 und AG Kamen HRB 0061 Berliner Commerzbank AG Berlin Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG Konto-Nr 2415008 Bankleitzahl 100 700 00 Postscheckamt Berlin West Konto-Nr 1175 101 Bankleitzahl 100 100 10

| Verbindungen | Wirkstoffkonzentration in % | Schlupfverhinderung in % |
|---|---|---|
| E-α-Methylthio-2-fluorzimtsäurenitril | 0,1 | 100 |
| Z-α-Methylthio-2-fluorzimtsäurenitril | 0,1 | 100 |
| α-Methylthio-4-methylzimtsäurenitril, Isomerengemisch | 0,1 | 100 |

-15-

Postanschrift: Schering Aktiengesellschaft, Postfach 65 03 11, D-1000 Berlin 65 · Fur Besucher Berlin-Wedding Mullerstraße 170-178 Telegramme: Scheringchemie Berlin

Vorstand Dr Herbert Asmis Dr Christian Bruhn, Dr Heinz Hannse Horst Kramp, Dr Klaus Pohle Dr Horst Witzel Vorsitzender des Aufsichtsrats Hans-Jürgen Hamann Sitz der Gesellschaft Berlin und Bergkamen Handelsregister AG Charlottenburg 93 HRB 283 und AG Kamen HRB 0061 Berliner Commerzbank AG, Berlin, Konto-Nr 108700600 Bankleitzahl 100 400 00 Berliner Handels- und Frankfurter Bank, Berlin Konto-Nr 70045224, Bankleitzahl 100 202 00 Deutsche Bank Berlin AG, Konto-Nr 2415008 Bankleitzahl 100 700 00 Postscheckamt Berlin West Konto-Nr 1175-101 Bankleitzahl 100 100 10

BEISPIEL 30

Ovizide Wirkung auf Eigelege der Ägyptischen Baumwolleule (Spodoptera littoralis)

Die erfindungsgemäß zu verwendenden Verbindungen sowie die Vergleichssubstanzen wurden als wäßrige Suspension beziehungsweise Emulsion mit den gewünschten Konzentrationen eingesetzt. In diese Wirkstoffzubereitungen wurden 1 Tag alte Eigelege, die von befruchteten Falterweibchen auf Filterpapier abgesetzt worden waren bis zur völligen Benetzung getaucht und für vier Tage in geschlossenen Petrischalen deponiert. Kriterium für die Wirkungsbeurteilung war die prozentuale Schlupfverhinderung im Vergleich zu unbehandelten Eigelegen.

Die erzielten Ergebnisse sind in nachstehender Tabelle zusammengefaßt.

| Verbindungen | Wirkstoffkonzentration in % | Schlupfverhinderung in % |
|---|---|---|
| α-Methylthio-zimtsäurenitril | 0,0064 | 100 |
| α-Methylthio-2-fluorzimtsäurenitril | 0,0064 | 95 |
| Vergleichsmittel gemäß US-Patent 3828091 | | |
| 2-(4-Chlorphenyl)-3-isopropylthio-acrylnitril | 0,0064 | 0 |
| 2-3-Butylthio-2-(4-Chlorphenyl)-acrylnitril | 0,0064 | 0 |
| 2-(4-Chlorphenyl)-3-äthylthio-acrylnitril | 0,0064 | 0 |

-16-

BEISPIEL 31

Ovizide Wirkung auf Eigelege des Mexicanischen Bohnenkäfers (Epilachna varivestis)

Die erfindungsgemäß zu verwendenden Verbindungen sowie die Vergleichssubstanzen wurden als wäßrige Suspensionen bzw. Emulsionen mit den gewünschten Konzentrationen eingesetzt. In diese Wirkstoffzubereitungen wurden 1 Tag alte Eigelege die von befruchteten Käferweibchen auf Primärblätter der Buschbohne (Phaseolus vulgaris) abgesetzt worden waren bis zur völligen Benetzung getaucht und für 6 Tage in geschlossenen Petrischalen deponiert. Kriterium für die Wirkungsbeurteilung war die prozentuale Schlupfverhinderung im Vergleich zu unbehandelten Eigelegen.

Die erzielten Ergebnisse sind in nachstehender Tabelle zusammengefaßt.

| Verbindungen | Wirkstoffkonzentration in % | Schlupfverhinderung in % |
|---|---|---|
| α-Methylthio-zimtsäurenitril | 0,04 | 95 |
| α-Methylthio-2-fluorzimtsäurenitril | 0,04 | 95 |
| α-Ethylthio-2-fluorzimtsäurenitril | 0,04 | 50 |
| Vergleichsmittel gemäß US-Patent 3 828 091 | | |
| 2-(4-Chlorphenyl)-3-Isopropylthioacrylnitril | 0,04 | 0 |
| 2-3-Butylthio-2-(4-chlorphenyl)-acrylnitril | 0,04 | 0 |
| 2-(4-Chlorphenyl)-3-ethylthioacrylnitril | 0,04 | 0 |

-17-

BEISPIEL 32

Nematizide Wirksamkeit auf $L_2$-Larven des Wurzelgallennematoden (Meloidogyne incognita)

Die erfindungsgemäß zu verwendenden Verbindungen wurden als pulverförmige Formulierungen suspendiert, das Vergleichsmittel lag als wäßrige Lösung vor. Aus Eimassen des Wurzelgallennematoden frisch geschlüpfte $L_2$-Larven wurden in die Lösungen eingegeben und nach 24 Stunden die Aktivität bzw. Inaktivität der Larven unter dem Mikroskop bestimmt. Die Ergebnisse sind der nachstehenden Tabelle zu entnehmen.

Meloidogyne-$L_2$ Larven, Einwirkungszeit 24 Stunden, Inaktivität in %

| Verbindungen | Wirkstoffkonzentration 0,01 | 0,005 | 0,0025 | 0,0012 | 0,0006 % |
|---|---|---|---|---|---|
| α-Methylthio-4-chlorzimtsäurenitril | 100 | 100 | 100 | 100 | 100 % |
| α-Methylthio-4-bromzimtsäurenitril | 100 | 100 | 100 | 100 | 100 % |
| α-Methylthio-4-fluorzimtsäurenitril | 100 | 100 | 100 | 100 | 95 % |
| Vergleichsmittel | | | | | |
| 1-(Dimethylaminocarbonyl)-N-(methylaminocarbonyloxy)-thioformhydroximsäure-methylester | 100 | 95 | 70 | 30 | 0 % |
| Unbehandelte Kontrolle | 0 | 0 | 0 | 0 | 0 % |

-18-

BEISPIEL 33

Ovizide Wirksamkeit auf Eimassen des Wurzelgallennematoden Meloidogyne incognita

Die erfindungsgemäß zu verwendenden Verbindungen wurden als pulverförmige Formulierungen suspendiert, das Vergleichsmittel lag als wäßrige Lösung vor. Von Capsicum anuum frisch gewonnene Eimassen wurden gewaschen und in die Lösung eingegeben. Nach einer Einwirkungsdauer von 48 Stunden folgte ein gründliches Waschen der behandelten Eimassen unter fließendem Wasser. Je Konzentration wurden 5 Eimassen in klares Wasser überführt und zum Larvenschlupf bei 24°C aufgestellt. Die nachfolgende Tabelle zeigt die Anzahl der geschlüpften $L_2$-Larven aus jeweils 5 Eimassen nach 8 Tagen.

Anzahl geschlüpfter Meloidogyne $L_2$-Larven aus behandelten Eimassen - Einwirkungsdauer 48 Stunden

| Verbindungen | Wirkstoffkonzentration 0,01 | 0,005 | 0,0025 | 0,0012 % |
|---|---|---|---|---|
| α-Methylthio-4-chlorzimtsäurenitril | 0 | 0 | 0 | 0 |
| α-Methylthio-4-bromzimtsäurenitril | 0 | 0 | 0 | 0 |
| α-Methylthio-4-fluorzimtsäurenitril | 0 | 0 | 0 | 50 |
| Vergleichsmittel | | | | |
| 1-(Dimethylaminocarbonyl)-N-(methylaminocarbonyloxy)-thioformhydroximsäure-methylester | 400 | 400 | 400 | 400 (zirka) |
| Unbehandelte Kontrolle | 400 | 400 | 400 | 400 (zirka) |

-19-

## BEISPIEL 34

Schlüpfhemmwirkung auf den Cysteninhalt von Heterodera schachtii

Die erfindungsgemäß zu verwendenden Verbindungen wurden als pulverförmige Formulierung suspendiert, das Vergleichsmittel lag als wäßrige Lösung vor. Cysten des Rübennematoden Heterodera schachtii wurden nach einer Einwirkungsdauer von 48 Stunden unter fließendem Wasser gründlich gewaschen und in Wurzeldiffusat bei 24°C zum $L_2$-Larvenschlupf angesetzt, je Konzentration 5 Cysten.
Die nachfolgende Tabelle zeigt die Anzahl geschlüpfter $L_2$-Larven aus jeweils 5 Cysten nach 8 Tagen.

| Verbindung | Wirkstoffkonzentration: 0,02 | 0,01 | 0,005 | 0,0025 % |
|---|---|---|---|---|
| α-Methylthio-4-chlorzimtsäurenitril | 0 | 0 | 0 | 0 |
| **Vergleichsmittel** | | | | |
| 1-(Dimethylaminocarbonyl)-N-(methylaminocarbonyloxy)-thioformhydroximsäure-methylester | 400 | 400 | 400 | 400 |
| Unbehandelte Kontrolle | 400 | 400 | 400 | 400 |

-20-

PATENTANSPRÜCHE

1. Mittel zur Bekämpfung von Schadorganismen, gekennzeichnet durch einen Gehalt an mindestens einem α-Alkylthiozimtsäurenitril der allgemeinen Formel

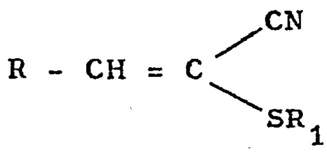
, I

in der

R einen gegebenenfalls ein- oder mehrfach, gleich oder verschieden durch Halogen, $(C_1-C_4)$-Alkyl, $(C_1-C_4)$-Alkoxy, $(C_1-C_4)$-Alkylthio oder Trifluormethyl substituierten aromatischen Kohlenwasserstoffrest und

$R_1$ einen gradkettigen $(C_1-C_4)$-Alkylrest oder den Cyanäthylrest bedeuten.

2. Mittel gemäß Anspruch 1, worin

R Phenyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Jodphenyl, 3-Jodphenyl, 4-Jodphenyl, 2,4-Difluorphenyl, 2,6-Difluorphenyl, Pentafluorphenyl, 2,4-Dichlorphenyl, 3,4-Dichlorphenyl, 2-Chlor-6-fluorphenyl, 2-Methylphenyl, 3-Methylphenyl, 4-Methylphenyl, 2-Methoxyphenyl, 3-Methoxyphenyl, 4-Methoxyphenyl, 2-Methylthiophenyl, 3-Methylthiophenyl, 4-Methylthiophenyl, 3-Trifluormethyl, 4-Trifluormethyl, 2-Pyridyl, 3-Pyridyl, 4-Pyridyl, 2-Thienyl, 3-Thienyl oder 1-Naphthyl und

$R_1$ Methyl, Äthyl, Propyl oder Butyl bedeuten.

3. Mittel gemäß Anspruch 1, gekennzeichnet durch einen Gehalt an α-Methylthio-zimtsäurenitril, α-Methylthio-2-fluorzimtsäurenitril, α-Methylthio-4-chlorzimtsäurenitril, α-Methylthio-4-bromzimtsäurenitril oder α-Methylthio-4-fluorzimtsäurenitril.



Postanschrift: Schering Aktiengesellschaft, Postfach 65 03 11, D-1000 Berlin 65 · Für Besucher Berlin-Wedding, Müllerstraße 170-178 Telegramme Scheringchemie Berlin
Vorstand Dr. Herbert Asmis Dr. Christian Bruhn Dr. Heinz Hannse, Horst Kramp Dr. Klaus Pohle, Dr. Horst Witzel Vorsitzender des Aufsichtsrats: Hans-Jürgen Hamann Sitz der Gesellschaft Berlin und Bergkamen Handelsregister AG Charlottenburg 93 HRB 283 und AG Kamen HRB 0061 · Berliner Commerzbank AG, Berlin, Konto-Nr 108700600 Bankleitzahl 100 400 00 Berliner Handels- und Frankfurter Bank Berlin Konto-Nr 70045224 Bankleitzahl 100 202 00 Deutsche Bank Berlin AG, Konto-Nr 2415008 Bankleitzahl 100 700 00 Postscheckamt Berlin West Konto-Nr 1175-101, Bankleitzahl 100 100 10

4. Mittel gemäß Anspruch 1 in Mischung mit Träger- und/oder Hilfsstoffen.

5. Mittel gemäß Anspruch 1 zur Bekämpfung von Insekten und Nematoden sowie deren Larven und Eiern.

6. Verfahren zur Herstellung von α-Alkylthiozimtsäurenitrilen der allgemeinen Formel I gemäß Anspruch 1, dadurch gekennzeichnet, daß man aromatische Aldehyde der allgemeinen Formel

$$R - CHO \qquad II$$

mit Alkylthioacetonitrilen der allgemeinen Formel

$CH_2 - SR_1$
|
$CN$

in Gegenwart von 18-crown-6 als Katalysator und unter Zusatz einer Base gegebenenfalls gelöst in einem Lösungsmittel kondensiert und das gewünschte Reaktionsprodukt in an sich bekannter Weise isoliert.



Postanschrift: Schering Aktiengesellschaft, Postfach 65 03 11, D-1000 Berlin 65 · Fur Besucher Berlin-Wedding, Müllerstraße 170-178 Telegramme: Scheringchemie Berlin
Vorstand Dr Herbert Asmis, Dr Christian Bruhn Dr Heinz Hannse Horst Kramp, Dr Klaus Pohle, Dr Horst Witzel Vorsitzender des Aufsichtsrats Hans-Jurgen Hamann
Sitz der Gesellschaft Berlin und Bergkamen Handelsregister, AG Charlottenburg 93 HRB 283 und AG Kamen HRB 0061 · Berliner Commerzbank AG, Berlin, Konto-Nr 108700600, Bankleitzahl 100 400 00 Berliner Handels- und Frankfurter Bank, Berlin Konto-Nr 70045224, Bankleitzahl 100 202 00 Deutsche Bank Berlin AG, Konto-Nr 2415008 Bankleitzahl 100 700 00 Postscheckamt Berlin West, Konto-Nr 1175-101 Bankleitzahl 100 100 10

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0131692
Nummer der Anmeldung
EP 84 10 3747

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | TETRAHEDRON LETTERS, Nr. 30, Juli 1978, Pergamon Press Ltd. Oxford, GB<br>F. POCHAT: "Voies d'accès aux nitriles thioéthers RR'-C=C(SEt)-CN", Seiten 2683, 2684<br>* Insgesamt *<br>-- | 6 | A 01 N 37/38<br>A 01 N 37/36<br>C 07 C 149/415<br>A 01 N 43/40<br>A 01 N 43/10<br>C 07 D 213/57<br>C 07 D 333/24 |
| X | SYNTHESIS, Nr. 5, Mai 1980 Stuttgart, DE<br>F. POCHAT: "2,4-Diaminopyrimidines: 6-Substituted 5-Alkylthio (or Arylthio) Derivatives", Seiten 379-381<br>* Seite 379, rechte Spalte, Tabelle *<br>-- | 6 | |
| X | CHEMICAL & PHARMACEUTICAL BULLETIN, Band 26, Nr. 6, Juni 1978 ./. | | **RECHERCHIERTE SACHGEBIETE (Int. Cl. 3)**<br>A 01 N<br>C 07 C |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1,3-6

Unvollständig recherchierte Patentansprüche: 2

Nicht recherchierte Patentansprüche:

Grund für die Beschränkung der Recherche: Gemäss Patentanspruch 1 bedeutet R der allgemeinen Formel I einen gegebenenfalls substituierten aromatischen Kohlenwasserstoffrest. Aber gemäss Patentanspruch 2 kann R auch 2-Pyridyl, 3-Pyridyl, 4-Pyridyl, 2-Thieryl oder 3-Thieryl bedeuten.

| Recherchenort | Abschlußdatum der Recherche | Prüfer |
|---|---|---|
| Den Haag | 03-10-1984 | FLETCHER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82

Europäisches Patentamt

**EUROPÄISCHER TEILRECHERCHENBERICHT**

0131692
Nummer der Anmeldung

EP 84 10 3747 -2-

| EINSCHLÄGIGE DOKUMENTE | | | KLASSIFIKATION DER ANMELDUNG (Int Cl 4) |
|---|---|---|---|
| Kategorie | Kennzeichnung des Dokuments mit Angabe, soweit erforderlich, der maßgeblichen Teile | betrifft Anspruch | |
| | Tokyo, JP<br>S. KANO et al.: "A Synthetic Application of Methylthioaceto-nitrile. I. A Synthesis of Cis- and trans-beta-Arylacrylonitriles, beta, omega-Unsaturated Nitriles and Carbonyl Compounds" Seiten 1874-1879<br><br>* Seite 1874, Zeile 12 - Seite 1876, Zeile 1; Seite 1878, Zeilen 8-14 *<br><br>-- | 6 | |
| | | | RECHERCHIERTE SACHGEBIETE (Int. Cl 4) |
| DA | US A - 3 828 091 (J.G. STRONG)<br><br>* Spalte 1, Zeile 51 - Spalte 2, Zeile 35; Spalte 7, Zeilen 9-30 ; Ansprüche *<br><br>----------- | 1-4 | |

EPA Form 1505.3 06.78